# EUROPEAN PATENT APPLICATION

(11) **EP 2 832 455 A1**
(43) Date of publication of application: **04.02.2015**
(21) Application number: 14179131.9
(22) Date of filing: 30.07.2014
(51) Int. Cl.: B06B 3/00, G01N 1/38, G01N 35/00

(54) **System and method for ultrasonic sample preparation**

(30) Priority: 01.08.2013 US 201313956547
(71) Applicant: Sonics & Materials Inc., Newtown CT 06470 (US)
(72) Inventor: Donaty, Michael, Danbury, CT Connecticut 06811 (US)
(74) Representative: Cabinet Laurent & Charras

(57) **Abstract**

A system and method for ultrasonic sample preparation, includes a vessel having a wall defining an inner volume. An ultrasonic probe is disposed in the inner volume of the vessel. A microplate having a plurality of sample wells is also disposed in the inner volume of the vessel. An actuator is connected to the microplate and is configured to move the microplate relative to the ultrasonic probe in the inner volume to facilitate uniform distribution of ultrasonic energy.

## Description

### FIELD OF THE INVENTION

The present invention relates to the use of ultrasonic energy to process materials. In particular, the present invention relates to a system and method for preparing sample materials using ultrasonic energy.

### BACKGROUND OF THE INVENTION

It is well known to use ultrasonic energy to prepare sample materials for diagnostic investigations. For example, Chromatin Immunoprecipitation (ChIP) assays, a type of immunoprecipitation experimental technique used to investigate the interaction between proteins and DNA in the cell, uses ultrasonic energy.

The CHIP technique involves: (1) crosslinking proteins with DNA; (2) fragmenting (also referred to as shearing) the chromatin (cross linked DNA-protein complex) from the cell; (3) preparing the soluble chromatin; (4) immunoprecipitating the protein of interest; and (5) identifying the segment associated with the protein. The fragmenting step is typically performed by transmitting ultrasonic energy to cells via an ultrasonic bath.

Typically, a sample material including chromatin is disposed in one or more sample wells of a microplate. A microplate is a flat plate with multiple "sample wells" used as small test tubes. A microplate may have, for example, 6, 24, 96, or 384 sample wells. Chromatin samples are disposed in one or more sample wells, and the microplate is immersed in a liquid received in a vessel. An ultrasonic probe is also disposed in the vessel. The probe is vibrated at a frequency, typically between 20 kHz and 40 kHz, thereby transmitting ultrasonic energy into the bath.

The transmission of the ultrasonic energy from the probe causes cavitation in the liquid in the vessel. Cavitation refers to the rapid formation and collapse of vapor pockets in a liquid in regions of very low pressure. In the case of ultrasonic mixing, the regions of very low pressure are formed by the rapid oscillation of the probe, i.e. the transmission of ultrasonic energy. The vapor pockets, or bubbles, quickly expand and contract. Cavitation of the liquid causes high levels of energy to be transmitted through the liquid. The cavitational energy within the bath is transmitted through the walls of the sample wells and to the chromatin samples disposed therein, thereby shearing the chromatin from the remainder of the cell in accordance with the CHIP technique.

This process has significant drawbacks, however. First, the energy transmitted into the liquid bath by the ultrasonic probe is not transmitted uniformly throughout the bath. As a result of this non-uniform dispersion of energy, the amount of ultrasonic energy received in each sample well varies.

Another disadvantage with the above described system and method for sample preparation is that the magnitude and duration of ultrasonic energy transmitted to different parts of the bath is unpredictable. Therefore, even if the non-uniform dispersion characteristics of the bath could be accounted for in a preparation protocol, the unpredictable nature of the energy transmission introduces unaccountable error.

Another disadvantage with the above described system and method is that the energy emitted by the probe is highly focused within the bath. As a result, sample wells proximate to probe may receive more ultrasonic energy, thus facilitating shearing therein, as compared to sample wells that are remote from the probe. As a result, the results of such assays are unreliable.

What is needed then, is a system and method for using ultrasonic preparation which minimizes the above-described drawbacks of traditional ultrasonic preparation.

### SUMMARY OF THE INVENTION

It is an object of the present invention to provide a system and method for ultrasonic sample preparation which avoids the problems of varying energy dispersion associated with the known systems and methods.

It is another object of the present invention to provide a system and method for ultrasonic sample preparation which avoids the problems of unpredictable energy transmissions.

These and other objects of the present invention are achieved by provision of a system for ultrasonic sample preparation. In one embodiment, the system includes a vessel having a wall defining an inner volume. An ultrasonic probe is disposed in the inner volume of the vessel. A microplate having a plurality of sample wells is also disposed in the inner volume of the vessel. An actuator is connected to the microplate and is configured to move the microplate relative to the ultrasonic probe in the inner volume.

In one embodiment of the present invention, the actuator is configured to move the microplate plate in a plane parallel to a surface of a liquid received in the inner volume.

In yet another embodiment of the present invention, the movement of the microplate relative to the ultrasonic probe facilitates uniform distribution of mechanical vibrations transmitted by the ultrasonic probe to each of the plurality of sample wells.

In yet another embodiment of the present invention, the actuator is configured to provide bidirectional movement of the microplate relative to the ultrasonic probe in the plane parallel to a surface of a liquid received in the inner volume.

In yet another embodiment of the present invention, the vessel wall defines a bottom of the vessel and at least a portion of the probe extends through the bottom of the vessel so that a portion of the probe which transmits mechanical vibrations is disposed in the inner volume.

In yet another embodiment of the present invention, the system includes a computer. Software executing on the computer generates a control signal indicative of a pattern of movement for the actuator such that each of the plurality of sample wells is positioned above the probe for at least the portion of a pattern of movement.

In yet another embodiment of the present invention, the system includes a carriage for supporting the microplate. In such embodiment, the actuator is connected to the microplate via the carriage.

In yet another embodiment of the present invention, a portion of the actuator is fixed relative to the wall of the vessel.

In yet a further embodiment of the present invention, a convertor is in communication with the ultrasonic probe. The convertor converts AC electricity to mechanical vibrations in the ultrasonic range.

In yet a further embodiment of the present invention, the microplate is selected from a group consisting of: a microplate having 6 sample wells, a microplate having 24 sample wells, a microplate having 96 sample wells, and a microplate having 384 sample wells.

In accordance with another aspect of the present invention, a method for ultrasonic sample preparation is disclosed. The method includes the steps of providing a vessel having a wall defining an inner volume. An ultrasonic probe is disposed in the inner volume of the vessel. A microplate having a plurality of sample wells is also disposed in the inner volume. Each of the sample wells has a sample disposed therein. The method further includes the step of providing an actuator. Mechanical vibrations are transmitted from the ultrasonic probe to the samples via a liquid received in the inner volume and via the microplate. The actuator moves the microplate in the inner volume relative to the ultrasonic probe.

In one embodiment, the method includes the step of moving the microplate in a plane parallel to a surface of a liquid received in the inner volume.

In yet another embodiment of the present invention, the step of moving the microplate relative to the ultrasonic probe facilitates uniform distribution of mechanical vibrations transmitted by the ultrasonic probe to each sample disposed in the plurality of sample wells.

In yet a further embodiment of the present invention, the method includes the step of moving the microplate relative to the ultrasonic probe includes bidirectional movement in a plane parallel to a surface of a liquid received in the inner volume.

In yet a further embodiment, the wall of the vessel defines a bottom of the vessel and at least a portion of the probe extends through the bottom of the vessel.

In yet a further embodiment of the present invention, the method includes the step of moving the microplate so that each of the plurality of sample wells is positioned above the probe for at least a portion of a pattern of movement.

In yet a further embodiment of the present invention, the method includes the step of providing a carriage for supporting the microplate.

In yet a further embodiment, the actuator is fixed relative to the wall of the vessel and is connected to the microplate.

In yet a further embodiment of the present invention, the method includes the step of providing a convertor which converts AC electricity to mechanical vibrations in the ultrasonic range. The convertor is in communication with the ultrasonic probe.

In yet a further embodiment, the microplate is selected from a group consisting of: a microplate having 6 sample wells, a microplate having 24 sample wells, a microplate having 96 sample wells, and a microplate having 384 sample wells.

The invention and its particular features and advantages will become more apparent from the following detailed description considered with reference to the accompanying drawings.

### BRIEF DESCRIPTION OF THE DRAWINGS

FIG. 1 illustrates a system for ultrasonic sample preparation in accordance with one embodiment of the present invention.
FIG. 2 illustrates a top view of a portion of the system shown in FIG. 1.
FIGS. 3A - 3F illustrate movement of the microplate relative to the ultrasonic probe in the inner volume of the vessel in accordance with one or more embodiments of the present invention.
FIG. 4 illustrates a method for ultrasonic sample preparation in accordance with one embodiment of the present invention.

### DETAILED DESCRIPTION OF THE INVENTION

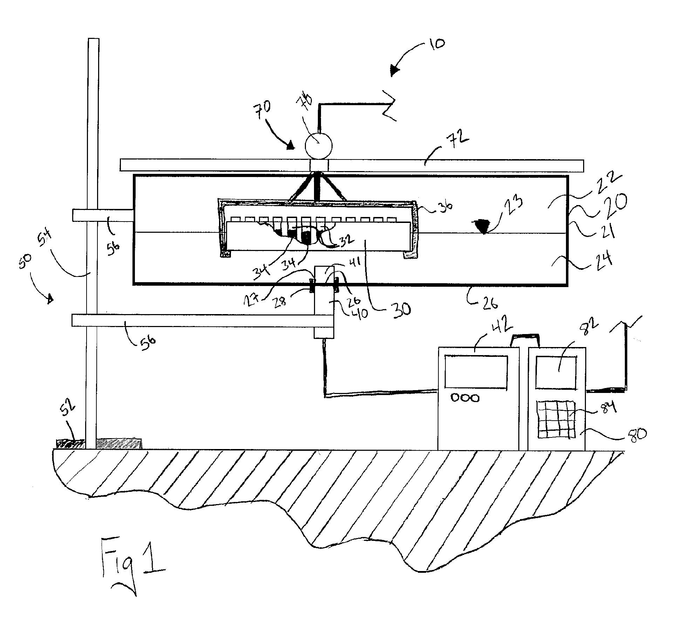

In reference to FIGS. 1-2, a system 10 for ultrasonic sample preparation is shown. The system 10 includes vessel 20. It should be noted that a front face of the vessel 20 is not shown in FIG. 1 in order to more fully illustrate the present invention through FIG. 1. The vessel 20 has a wall 21 that defines an inner volume 22. The inner volume 22 is configured such that a liquid 24 can be received and contained therein. In FIG. 1, liquid water 24 is received in the inner volume 22. It should be understood, however, that one or more different liquids may be received in the inner volume 22 of the vessel 20. The liquid 24 facilitates transmission of mechanical vibrations emitted from an ultrasonic probe 40 (discussed in detail below) to a sample 34 (discussed in detail further below).

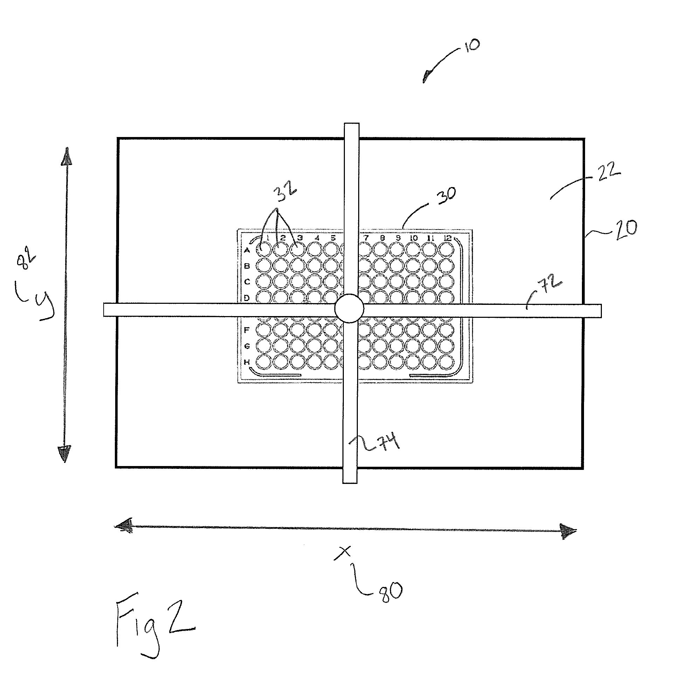

The system 10 includes a microplate 30 disposed in the inner volume 22 of the vessel 20. The microplate 30, also known as a Microtitre plate or microwell plate, is a flat plate with multiple "sample wells" 32 which can serve as small test tubes. In reference to the CHIP assay example described in the Background Section, chromatin samples 34 may be disposed in one or more of the sample wells 32. In reference to the view of the microplate 30 shown in FIG. 1, a portion of a sidewall is cutaway for the purpose of illustrating some of the sample wells 32. Each of the sample wells 32 defines an inner volume and is configured to receive and hold a liquid, for example, a sample 34. In reference to FIG. 2, the microplate 30 includes ninety-six sample wells 32. It should be understood, however, that the number of sample wells 32 in a microplate 30 for use with the present invention can vary and is not limited to 96.

Microplates 30 for use with the present invention typically have 6, 24, 96, 384, or more, sample wells 32. The sample wells 32 are generally arranged in a 2:3 rectangular matrix. For example, in reference to the microplate 30 shown in FIG. 2, the microplate 30 has ninety-six sample wells 32 arranged in rows A-H and in columns 1 - 12. In the embodiment shown 10, the microplate 30 is manufactured from polystyrene using an injection molding method. The present invention is not limited in this regard as the microplate 30 can be manufactured from a variety of materials using any known method of manufacture. In practice samples 34 (shown in FIG. 1) to be prepared during a protocol are received sample wells 32 of the microplate 30. The samples 34 included in the microplate 30 may be the same, may be different, or may include a combination or similar and dissimilar samples.

An ultrasonic probe 40 is disposed in the inner volume 22 of the vessel 20. As shown in FIG. 1, a portion of the ultrasonic probe 40 extends through an aperture 27 in a bottom wall 26 of the vessel 20. In the embodiment shown, the aperture 27 is sized such that a cross-sectional area thereof is similar to a cross-sectional area of the ultrasonic probe 40 extending through the aperture 27, thereby inhibiting leakage of the fluid 24 from the inner volume 22 of the vessel 20. In the embodiment shown, a seal 28 is provided between the ultrasonic probe 40 and a perimeter of the aperture 27 to further inhibit leakage of fluid 24.

The system 10 includes a converter 42 which converts electrical power from an electrical power source to ultrasonic energy. The ultrasonic probe 40 is coupled to the converter 42. The ultrasonic probe 40, and more specifically a distal end thereof, can be vibrated in the ultrasonic range, such as, for example, in the frequency range from about 20 kHz to about 40 kHz. Since ultrasonic sample preparation is well known, the operation and configuration of converter 42 and probe 40 is not discussed in detail herein. It should be noted, however, that although the system 10 shown in FIGS. 1-2 discloses the probe 40 extending through an aperture 27 in a bottom wall 26 of the vessel 20, the present invention is not limited in this regard. The probe 40 may, for example, extend through an aperture in a sidewall of the vessel 20, or, for example, the probe 40 may extend through an open or closed top of the vessel 20.

In the embodiment shown, the vessel 20 and the ultrasonic probe 40 are fixed relative to each other via a rigid stand 50. The stand 50 includes a base 52 and a support column 54 extending vertically therefrom. One or more support arms 56 extend horizontally from the support column 52, and are configured to receive and support one or more of the ultrasonic probe 40 and the vessel 20. The components of the stand 50 can be releaseably adjusted to one or more of the vessel 20 and the probe 40 to facilitate configuration of the system 10 for different protocols. It should also be understood that although a specific embodiment of a stand 50 is shown in FIGS. 1 - 2, the present invention is not limited in this regard as many different configurations of assembling and supporting the one or more of the probe 40 and vessel 20 may be used.

In reference to FIG. 1, the microplate 30 is connected to an actuator 70 via a carriage 36. The carriage 36 is configured to support the microplate 30 in the liquid 24 received in the inner volume 22. In the embodiment shown, the carriage 36 is configured to support at least two sides of the microplate 30. The carriage 36 is connected to an actuator 70, which is configured to move the microplate 30 in the inner volume 22 of the vessel 20. It should be understood, however, that many different arrangements may be employed and the present invention is not limited in this regard. For example, in one embodiment, the actuator 70 is connected directly to the microplate 30 and there is no separate carriage 36 associated with the system 10.

In the embodiment shown, the actuator 70 is illustrated by first 72 and second 74 supports. The first support 72 extends in a first direction across a first length of the vessel 20. The second support 74 extends in a second direction across a second length of the vessel 20, the second direction being perpendicular to the first direction in a plane parallel to a surface 23 of the liquid 24 received in the inner volume 22. The actuator 70 is configured to provide bidirectional movement of the carriage 36, and as a result the microplate 30, in the plane 23 parallel to the surface of the liquid 24 received in the inner volume 22. Since mechanical actuation is well known, the type of and configuration of the actuator 70 is not discussed in detail herein. However, operation of the actuator 70, as it pertains to the present invention, is discussed in detail below.

As shown in FIG. 1, the microplate 30 is disposed in the inner volume 22 and is supported by the actuator 70 via the carriage 36 so that a portion of the microplate 30 is below the surface 23 of the liquid 22. In the embodiment shown, the microplate 30 is disposed in the liquid 24 such that each sample received in a sample well 32 is below the surface 23 of the liquid 24. This ensures that the samples 34 received in the sample wells 32 are exposed to adequate ultrasonic energy via the liquid 24 during a testing or preparation protocol. It should be understood, however, that the present invention is not limited in this regard and that the only a portion of the microplate must be below the surface 23 in order to facilitate transmission of the ultrasonic energy to the samples 34. In the embodiment shown in FIGS. 1-2, the microplate 30 is fixed vertically via the carriage 36 and actuator 70 relative to the vessel 20. It should be understood, however, that the present invention is not limited in this regard and that in some embodiments, the actuator 70 can move the microplate 30 up or down relative to the vessel 20.

As illustrated in FIG. 2, the actuator 70 can move the microplate 30 relative to the probe 40 in a first direction, identified by the position variable (x) 80. The actuator 70 can move the microplate 30 relative to the probe 40 in a second direction, identified by the position variable (y) 82. The actuator 70 is further configured to provide simultaneous movement of the microplate 30 in the first direction (x) and the second direction (y). In this manner, the actuator 70 is configured to provide bidirectional movement of the microplate 30 in a plane parallel to a surface 23 of a liquid 24 received in the inner volume 22.

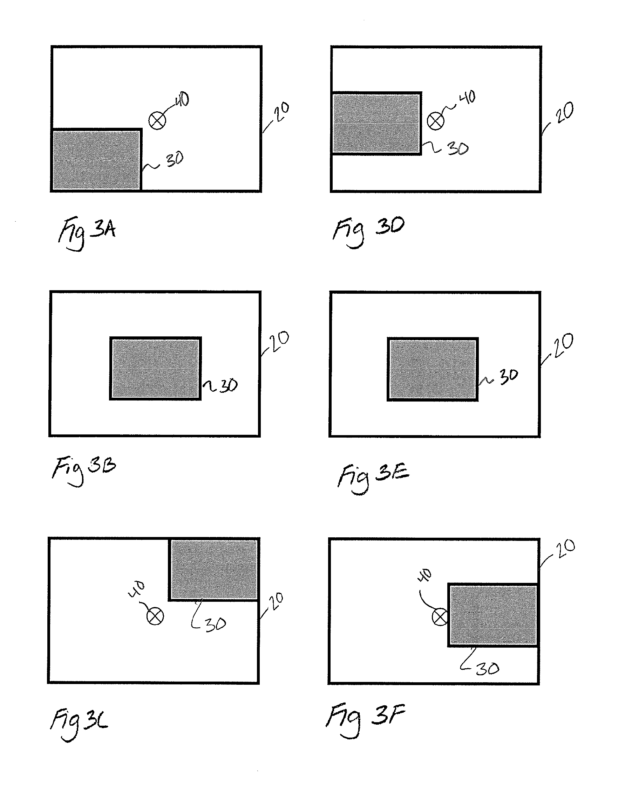

The movement of the microplate 30 relative to the ultrasonic probe 40 facilitates distribution of mechanical vibrations transmitted by the ultrasonic probe to each of the plurality of sample wells 32. In reference to FIGS. 3A - 3F, a range of possible movement of the microplate 30 relative to the probe 40 is illustrated. In FIG. 3A, the microplate 30 is shown in a lower left of the plane parallel to the 23 surface of the liquid 24 (the "horizontal plane"). The probe 40 is shown outside of the outline of the microplate, illustrating that neither the microplate, nor any of its sample wells 32, are above the ultrasonic probe 40. In FIG. 3B, the actuator 70 (not shown in FIG. 3A-3F) has moved the microplate 30 to the center of the vessel 20 in the horizontal plane so that at least a portion of the microplate is above the ultrasonic probe 40. In FIG. 3C, the actuator 70 has moved the microplate 30 to the upper right of the vessel 20 in the horizontal plane. Again, the ultrasonic probe 40 is visible because the microplate 30 is not directly above the ultrasonic probe.

In reference to FIG. 3D, the microplate 30 is positioned on the left side of the vessel 20 between the top and bottom thereof in the horizontal plane. In reference to FIG. 3E, the actuator 70 has moved the microplate 30 over the probe 40 to a center of the horizontal plane. In FIG. 3F, the actuator 70 has moved the microplate to the far right of the vessel in the horizontal plane. FIGS. 3A - 3C illustrate a range of bidirectional movement and FIGS. 3D - 3F illustrate a range of unidirectional movement. It should be understood to a person having ordinary skill in the art that many different combinations of movement, including both unidirectional and bidirectional movement, in the horizontal plane can be achieved using the actuator 70 in accordance with the present invention.

During a sample preparation protocol, the microplate 30 can be moved relative to the actuator 70 in the plane parallel to a surface 23 of a liquid 24 received in the inner volume to facilitate uniform distribution of mechanical vibrations transmitted by the ultrasonic probe 40 to each of the plurality of sample wells 32. In this manner, it is possible to ensure that each of the sample wells 32 receives a more uniform exposure to mechanical energy from the probe 40 as a compared to currently known systems and methods. Although the system 10 is disclosed with a single actuator 70, the present invention is not limited in this regard. For example, movement of the microplate 30 relative to the probe 40 can be accomplished using a plurality of actuators 70. In yet other embodiments, a single actuator 70 may include a plurality of motors 76 to accomplish movement of the microplate 30 relative to the probe 40. In yet further embodiments of the present invention, one or more actuators 70 are provided to effect movement of the vessel 20 and probe 40 relative to the microplate 30.

In the embodiment shown in FIGS. 1-2, the actuator is in communication with a controller 80 for generating and transmitting command signals for controlling the actuator 70 and motor 76 associated therewith. Thus, the controller 80 can be used to control movement of the microplate 30 relative to the probe 40 within the vessel 20. The controller 80 has one or more processors having software executing thereon for controlling the actuator 70. The controller 80 includes an interface 82, 84 for receiving commands regarding the movement of the actuator, and for displaying information relevant thereto. In some embodiments of the present invention, a control routine can be received and/or stored by the controller 80 or a database therewith. In certain embodiments, the controller 80 may store one or more control routines. In yet further embodiments, the controller includes software for executing thereon that develops a control routine based on one or more parameters input into the controller, for example, by an operator. For example, in one embodiment, an operator may request a control routine for a 96 sample well microplate. Software executing on the controller 80 will develop a control routine such that each of the sample wells will be exposed to substantially the same amount of mechanical energy from the probe. In yet another example, the operator may specify use of a 96 sample well microplate, however, the operator may indicate that only sample wells in columns 1 - 6 have samples disposed therein, while sample wells in columns 7 - 12 are empty. Software executing on the controller will generate a control routine that accounts for the specific pattern of samples received in the microplate.

During a testing or preparation protocol, software executing on the controller 80 generates instructions indicative of a position and/or movement of the actuator 70, and therefore the microplate 30, relative to the probe 40 based on a selected control routine. In this manner, it is possible to develop and execute precise movement of the microplate 30 relative to the probe 40 to facilitate uniform distribution of mechanical energy to each of the sample wells 32. It should be understood that although a controller 80 is shown, the present invention is not limited in this regard and some other type of method or system may be used to control the actuator 70. Similarly, although a specific embodiment of a controller 80 is shown, the present invention is not limited in this regard. For example, a control routine may be received directly by a control architecture associated with the motor 76. Or, for example, the control routine may be associated with a personal computer or the like that is in communication with the actuator 70.

In accordance with one embodiment of the present invention, one or more of the frequency and the magnitude of the ultrasonic vibrations emitted by the probe 40 can be adjusted via the convertor 42 to further facilitate uniform distribution of mechanical energy from the ultrasonic probe 40 to each of the sample wells 32 in the microplate 30. In the embodiment shown, the controller 80 is in communication with the convertor 42. As a result, it is possible to store and execute a control routine in which a position of the microplate 30 is moved relative to the probe 40 and in which one or more of the frequency and magnitude of the ultrasonic energy being emitted from the probe 40 is adjusted.

It should be understood that although the disclosed embodiment shows that the actuator 70 can move the microplate 30 entirely past the probe in both directions, for example as illustrated in FIGS. 3A-F, the present invention is not limited in this regard. For example, as will be appreciated by a person having ordinary skill in the art, the actuator 70 may adjust the position of the microplate 30 relative to the probe 40 so that the probe remains below the microplate at all times. It should be further understood that while the system includes a specific configuration of components, the present invention is not limited in this regard. For example, a person having ordinary skill in the art will appreciate that many different configurations and types of equipment may be employed to practice the presently disclosed invention.

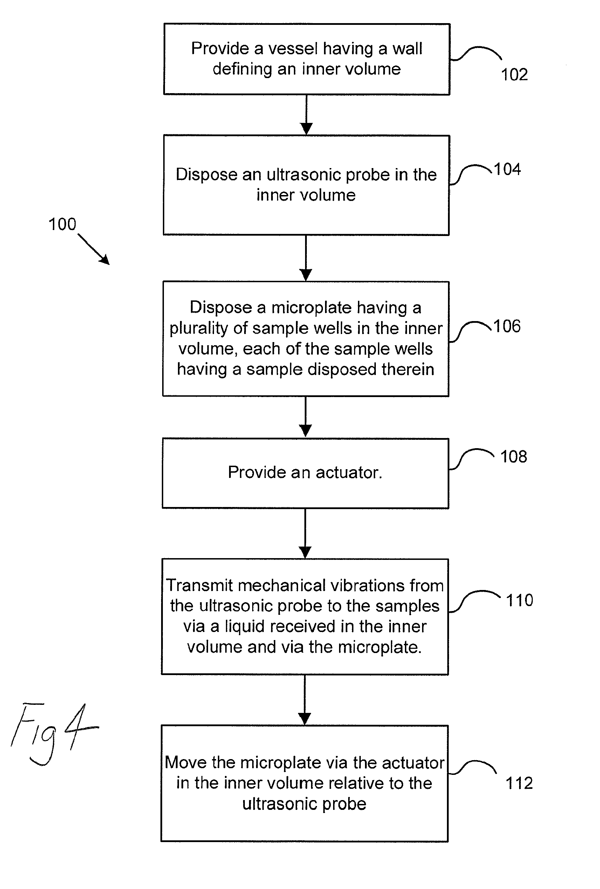

FIG. 4 illustrates a method 100 in accordance with one embodiment of the present invention. First, a vessel is provided having a wall defining an inner volume (step 102). Second, an ultrasonic probe is disposed in the inner volume of the vessel (step 104). Next, a microplate having a plurality of sample wells is disposed in the inner volume (step 106). Each of the sample wells has a sample disposed therein. Next an actuator is provided (step 108). Next, mechanical vibrations are transmitted from the ultrasonic probe to the samples via a liquid received in the inner volume and the microplate (step 110). Next, the microplate is moved via the actuator in the inner volume relative to the ultrasonic probe (step 112).

Although the invention has been described with reference to a particular arrangement of parts, features and the like, these are not intended to exhaust all possible arrangements or features, and indeed many other modifications and variations will be ascertainable to those of skill in the art.

## Claims

1. A system for ultrasonic sample preparation comprising:
a vessel having a wall defining an inner volume;
an ultrasonic probe disposed in the inner volume;
a microplate having a plurality of sample wells, the microplate disposed in the inner volume;
an actuator connected to the microplate and configured to move the microplate relative to the ultrasonic probe in the inner volume.

2. The system of claim 1, wherein the actuator is configured to move the microplate in a plane parallel to a surface of a liquid received in the inner volume.

3. The system of claim 2, wherein the movement of the microplate relative to the ultrasonic probe facilitates uniform distribution of mechanical vibrations transmitted by the ultrasonic probe to each of the plurality of sample wells.

4. The system of claim 3, wherein the actuator is configured to provide bidirectional movement of the microplate relative to the ultrasonic probe in the plane parallel to the surface of the liquid received in the inner volume.

5. The system of claim 2, wherein the wall of the vessel defines a bottom of the vessel and at least a portion of the probe extends through the bottom of the vessel so that a portion of the probe which transmits mechanical vibrations is disposed in the inner volume.

6. The system of claim 5, further comprising:
a computer;
software executing on the computer for generating a control signal indicative of a pattern of movement for the actuator such that each of the plurality of sample wells is positioned above the probe for at least a portion of a pattern of movement.

7. The system of claim 1, further comprising:
a carriage supporting the microplate;
wherein the actuator is connected to the microplate via the carriage.

8. The system of claim 7, wherein a portion of the actuator is fixed relative to the wall of the vessel.

9. The system of claim 2, further comprising:
a convertor which converts AC electricity to mechanical vibrations in the ultrasonic range, the convertor in communication with the ultrasonic probe.

10. The system of claim 2, wherein the microplate is selected from a group consisting of: a microplate having 6 sample wells, a microplate having 24 sample wells, a microplate having 96 sample wells, and a microplate having 384 sample wells.

11. A method for ultrasonic sample preparation, comprising the steps of:
providing a vessel having a wall defining an inner volume;
disposing an ultrasonic probe in the inner volume;
disposing a microplate having a plurality of sample wells in the inner volume, each of the sample wells having a sample disposed therein;
providing an actuator;
transmitting mechanical vibrations from the ultrasonic probe to the samples via a liquid received in the inner volume and via the microplate;
moving the microplate via the actuator in the inner volume relative to the ultrasonic probe.

12. The method of claim 11, wherein the microplate is moved in a plane parallel to a surface of a fluid received in the inner volume.

13. The method of claim 12, wherein the step of moving the microplate relative to the ultrasonic probe facilitates uniform distribution of mechanical vibrations transmitted by the ultrasonic probe to each sample disposed in the plurality of sample wells.

14. The method of claim 13, wherein the movement of the microplate relative to the ultrasonic probe is bidirectional in the plane parallel to the surface of the liquid received in the inner volume.

15. The method of claim 13, wherein the wall of the vessel defines a bottom of the vessel and at least a portion of the probe extends through the bottom of the vessel.
